# FASCICULE DE BREVET EUROPEEN

(11) **EP 0 673 934 B1**
(45) Date de publication et mention de la délivrance du brevet: **25.04.2001**
(21) Numéro de dépôt: 95400614.4
(22) Date de dépôt: 21.03.1995
(51) Int. Cl.: C07D 277/68, A61K 31/425

(54) **Nouvelles aminoalkyl benzothiazolinones, leur procédé de préparation et les compositions pharmaceutiques qui les contiennent**
Aminoalkyl Benzothiazolinone, Verfahren zu ihrer Herstellung und pharmazeutische Zusammensetzungen die sie enthalten
Aminoalkyl benzothiazolinones, process for their preparation and pharmaceutical compositions containing them

(30) Priorité: 22.03.1994 FR 9403299; 22.03.1994 FR 9403300
(43) Date de publication de la demande: 27.09.1995
(73) Titulaire: ADIR ET COMPAGNIE, 92415 Courbevoie Cédex (FR)
(72) Inventeur: Ait Mansour, Hamid, I-59100 Roubaix (FR); Taverne, Thierry, F-62222 Saint Martin Les Boulogne (FR); Houssin, Raymond, F-59700 Marq en Baroeul (FR); Lesieur, Isabelle, F-59147 Gondecourt (FR); Depreux, Patrick, F-59280 Armentieres (FR); Adam, Gérard, F-78600 Le Mesnil le Roi (FR); Caignard, Daniel-Henri, F-75015 Paris (FR); Renard, Pierre, F-78000 Versailles (FR); Rettori, Marie-Claire, F-92400 Courbevoie (FR)

(56) Documents cités:
- EP-A- 0 281 309
- EP-A- 0 366 511
- EP-A- 0 385 848
- EP-A- 0 478 446
- FARMACO, EDIZIONE PRATICA, vol. 44,no. 1, Janvier 1989 PAVIA IT, pages 77-88, Z.MOUSSAVI ET AL '(arylpiperazino-4-butyryl)-6 benzoxazolinones et produits de reduction:etude chimique et pharmacodynamique'
- JOURNAL OF MEDICINAL CHEMISTRY, vol. 34,no. 6, Juin 1991 WASHINGTON US, pages 1860-1866, JOHN A.LOWE III ET AL '1-Naphthylpiperazine derivatives as potential atypical antipsychotic agents'

## Description

La présente invention concerne de nouvelles aminoalkyl benzothiazolinones, leur procédé de préparation et les compositions pharmaceutiques qui les contiennent.

La demande de brevet EP 478446 décrit des dérivés (aminoalkyl)benzothiazolinones en tant qu'agents antipsychotiques, analgésiques et anxiolytiques.

La demanderesse a présentement découvert de nouvelles aminoalkyl benzothiazolinones qui possèdent de façon surprenante une affinité pour les récepteurs sigma (σ) beaucoup plus intense que les dérivés de la demande EP 478446.

La très haute affinité des dérivés de l'invention pour les récepteurs sigma σ, nettement supérieure à celle obtenue avec les dérivés de la demande EP 478446 les rend utilisables dans la prévention et le traitement des maladies liées à ce type de récepteurs, à savoir les maladies psychiatriques, la psychose, la schizophrénie, la dépression, le stress, l'anxiété, l'insuffisance circulatoire cérébrale, les troubles de la mémoire et la maladie d'Alzheimer, ainsi que les maladies inflammatoires de type immunitaire, l'arthrite aiguë ou l'arthrite chronique et les troubles du péristaltisme intestinal.

Par ailleurs la haute sélectivité des dérivés de la présente invention pour les récepteurs sigma, en particulier leur absence d'affinité pour les récepteurs D₂, permet leur utilisation en thérapeutique avec une sécurité accrue. En particulier, les effets secondaires de type extrapyramidaux que l'on rencontre lors du traitement avec des produits à forte composante D₂, à laquelle ces effets sont imputés, ne se retrouvent pas avec les produits de l'invention qui sont en moyenne 100 à 1000 fois moins affins pour les récepteurs D₂ que les dérivés de la demande EP 478446. Au bilan les produits de la présente invention présentent un rapport de sélectivité (affinité récepteurs sigma) : (affinité récetpeurs D₂) de 10 000 à 100 000 fois supérieur à celui obtenu avec les dérivés de la demande EP 478446 ce qui est tout à fait inattendu au vu de la demande EP 478 446, ce qui améliore considérablement leur sécurité d'emploi.
Plus particulièrement, la présente invention concerne les composés de formule (I) : 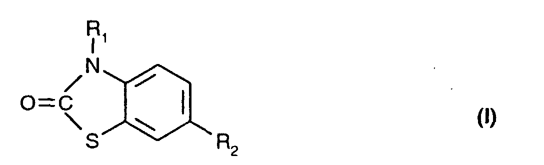 dans laquelle :
- R₁ représente un hydrogène ou un radical alkyle,
- et R₂ représente un groupement de formule R₂₁ : 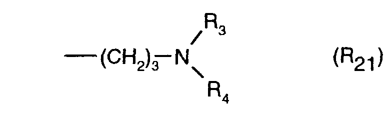
dans laquelle :
- R₃ et R₄ représentent indépendamment l'un de l'autre un atome d'hydrogène ou un radical choisi parmi alkyle, cycloalkyle, cycloalkylalkyle, alkényle, alkynyle, et -(CH₂)m aryle ; avec m représentant 0, 1, 2, 3 ou 4, le groupement aryle pouvant être non substitué ou substitué,
   ou bien R₃ et R₄ forment ensemble un hétérocycle saturé de 5 à 9 sommets pouvant contenir d'autres hétéroatomes choisis parmi oxygène et soufre et pouvant être non substitué ou substitué ou un groupement 4-(méthoxyphényl)-pipérazin-1-yl,
   ou groupement de formule R₂₂ : 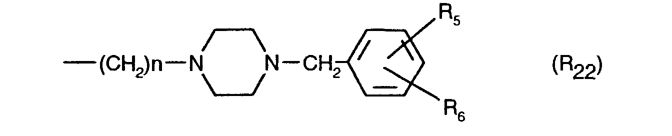 dans laquelle n représente 2,3 ou 4,
   et R₅ et R₆, identiques, représentent un hydrogène ou un halogène,
   étant entendu que lors de la description de la formule (I),
- le terme "substitué" associé au radical "aryle" signifie que la substitution peut se faire par un ou plusieurs radicaux choisis parmi halogène, hydroxy, alkyle, alkoxy inférieur et alkyle substitué par un ou plusieurs halogènes,
- le terme "substitué" associé à l'hétérocycle formé par -NR₃R₄ signifie que la substitution peut se faire par un ou plusieurs radicaux alkyle,
- les termes "alkyle" et "alkoxy" désignent des groupements linéaires ou ramifiés contenant de 1 à 6 atomes de carbone,
- les termes "alkényle" et "alkynyle" désignent des groupements linéaires ou ramifiés de 2 à 6 atomes de carbone,
- le terme "aryle" signifie phényle ou naphtyle,
- et le terme "cycloalkyle" représente un groupement cyclique de 3 à 8 atomes de carbone,
leurs isomères optiques et leurs sels d'addition à un acide pharmaceutiquement acceptable ou, lorsque R₁ représente un hydrogène, leurs sels d'addition à une base pharmaceutiquement acceptable.

L'invention concerne particulièrement les composés de formule (I) dans laquelle R₂ représente un groupement de formule R₂₁ et R₃ et R₄ forment ensemble avec l'atome d'azote qui les porte un groupement choisi parmi morpholino, thiomorpholino, pyrrolidino, pipéridino et perhydroazépino, par exemple morpholino.

L'invention concerne particulièrement les composés de formule (I) dans laquelle R₂ représente un groupement de formule R₂₂ et R₅ et R₆, identiques, représentent un chlore.

Parmi les acides pharmaceutiquement acceptables que l'on peut utiliser pour former un sel d'addition avec les composés de l'invention, on peut citer, à titre d'exemples et de façon non limitative, les acides chlorhydrique, sulfurique, phosphorique, tartrique, malique, maléique, fumarique, oxalique, méthanesulfonique, éthanesulfonique, camphorique, et citrique.

Parmi les bases pharmaceutiquement acceptables que l'on peut utiliser pour former un sel d'addition avec les composés de l'invention, on peut citer à titre d'exemples et de façon non limitative, les hydroxydes de sodium, de potassium, de calcium, ou d'aluminium, les carbonates de métaux alcalins ou alcalinoterreux, et les bases organiques comme la triéthylamine, la benzylamine, la diéthanolamine, la tert-butylamine, la dicyclohexylamine, et l'arginine.

De façon particulière, les radicaux alkyls présents dans la formule (I) peuvent être choisis parmi méthyl, éthyl, n-propyl, isopropyl, n-butyl, isobutyl, sec-butyl, tert-butyl, pentyl, ou hexyl.

Les radicaux alkoxy présents dans la formule (I) peuvent être choisis parmi méthoxy, éthoxy, n-propoxy, isopropoxy, n-butoxy, iso-butoxy, sec-butoxy, tert-butoxy, pentyloxy, et hexyloxy.

Les halogènes présents dans la formule (I) peuvent être choisis parmi le brome, le chlore, le fluor, et l'iode.

Les cycloalkyles présents dans les substituants de la formule (I) peuvent être choisis parmi cyclopropyle, cyclobutyle, cyclopentyle, cyclohexyle, cycloheptyle et cyclooctyle.

L'invention s'étend également au procédé de préparation des composés de formule (I) dans lequel on introduit dans une solution appropriée un composé de formule (II) : 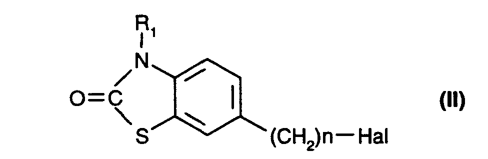 dans laquelle R₁ et n sont tels que définis dans la formule (I) et Hal est un atome d'halogène, que l'on condense :
- soit avec un composé de formule (III/a) : 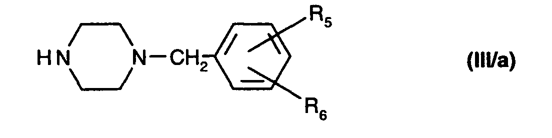 dans laquelle R₅ et R₆ sont tels que définis dans la formule (I),
   afin d'obtenir les composés de formule (I/a) : 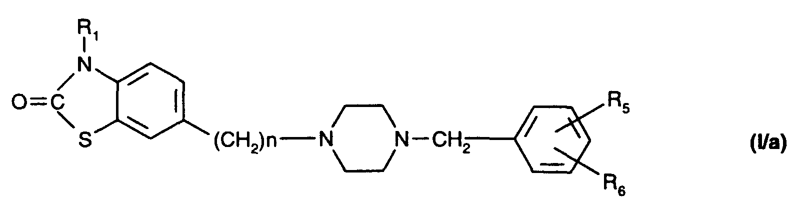 dans laquelle R₁, R₅, R₆ et n sont tels que définis précédemment,
- soit, lorsque n est égal à 3, avec un composé de formule (III/b) : 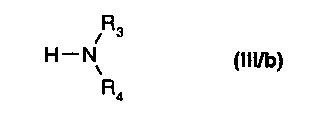 dans laquelle R₃ et R₄ sont tels que définis dans la formule (I),
   pour obtenir un composé de formule (I/b) : 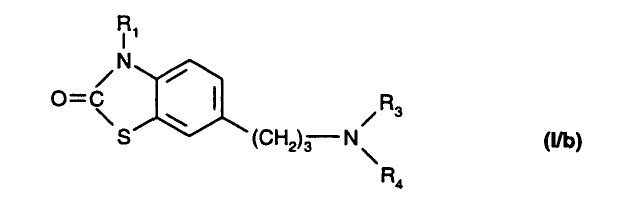 dans laquelle R₁, R₃ et R₄ sont tels que définis précédemment,
les composés de formule (I/a) et (I/b) formant l'ensemble des composés de formule (I), composés de formule (I) qui sont, si on le désire, séparés, le cas échéant, en leurs différents isomères optiques ou salifiés avec un acide pharmaceutiquement acceptable, ou si R₁ représente un atome d'hydrogène, salifiés par une base pharmaceutiquement acceptable.

Les matières premières utilisées dans le procédé précédemment décrit sont soit commerciales, soit aisément accessibles à l'homme du métier selon des procédés bien connus dans la littérature. On se réfèrera plus particulièrement, pour les composés de formule (II), à la description de la demande de brevet EP 430 800.
Les composés de formule (I) possèdent des propriétés pharmacologiques intéressantes.

La très haute affinité des dérivés de l'invention pour les récepteurs σ les rend utilisables dans le traitement des désordres moteurs, les dystonies (Walker, JM : Drug specificity of pharmacology dystonia, Pharmacol. Biochem. Behav. 1990, 36, 151), la dyskinésie tardive (Lindstrom, L.H. : Acta Psychiatr. Scand. 1988, 77, 1122), les troubles psychotiques (Chouinard, F., Annable, L. Psychopharmacology 1984, 84, 282), et dans le traitement des dommages liés à l'ischémie périphérique ou cérébrale, de l'insuffisance circulatoire cérébrale, des troubles de mémoire, de la maladie d'Alzheimer et des états de choc (Pontecorvo, M.J., Brain Res. Bull. 1991, 26, 461), dans la régulation des phénomènes immunitaires (Carroll, F.I., Med. Chem. Res. 1992, 2, 3), le traitement des toxicomanies à la cocaïne (Abou - Gharbia, M., Academic. Press. Inc. Bristol. J. Ed. Publisher. 1993, 28, 1), le diagnostic et la localisation des tumeurs (Hudzik, T.J., Psychopharmacology. 1992, 108, 115 ; Abou - Gharbia, M., Academic. Press. Inc. Bristol. J. Ed. Publisher 1993, 28, 1) et le vomissement (Hudzik, T.J., Eur. J. Pharmacol. 1993, 236, 279), ainsi que dans le traitement des maladies inflammatoires d'origine immunitaire et des troubles de la motricité intestinale.

La présente invention a également pour objet les compositions pharmaceutiques contenant les composés de formule (I) ou un de leurs sels d'addition à un acide ou le cas échéant à une base pharmaceutiquement acceptable, en combinaison avec un ou plusieurs excipients.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,1 mg et 1 g par 24 heures en 1 ou 2 prises, plus particulièrement 1 à 100 mg, par exemple 1 à 10 mg.

Les exemples suivants illustrent l'invention.

Les spectres de résonance magnétique nucléaire ¹H ont été réalisés en utilisant le TMS (tétraméthylsilane) comme référence interne. Les déplacements chimiques sont exprimés en partie par million (p.p.m). Les spectres infrarouge ont été effectués sous forme de pastille de bromure de potassium renfermant environ 1 % du produit à analyser.

Les préparations ne font pas partie de l'invention mais sont utiles pour réaliser la synthèse des dérivés de l'invention.

### PREPARATION 1 : 6-(3-CHLOROPROPIONYL)BENZOTHIAZOLINONE

### Réactifs :

| | |
|---|---|
| Benzothiazoline | 0,05 mole (7,55 g) |
| Chlorure de 3-chloropropionyle | 0,05 mole (4,77 cm³) |
| Chlorure d'aluminium | 0,40 mole (52,50 g) |
| Diméthylformamide anhydre | 0,12 mole (9,20 cm³) |

### Mode opératoire :

Dans une fiole à col rodé surmontée d'un réfrigérant à eau, introduire 0,40 mole de chlorure d'aluminium puis, goutte à goutte et sous agitation magnétique, 0,12 mole de diméthylformamide anhydre.

Ajouter 0,05 mole de benzothiazolinone et stabiliser la température à 70°C. Bien homogénéiser le milieu et ajouter lentement 0,05 mole de chlorure de 3-chloropropionyle.

Après addition, laisser une heure sous agitation à 70°C. Verser le milieu sur de la glace pilée, essorer le précipité formé, le laver à l'eau jusqu'à neutralité des eaux de lavage, le sécher puis le recristalliser.

| | |
|---|---|
| Masse moléculaire | 241,69 g.mole⁻¹ pour C₁₀H₈ClNO₂S |
| Point de fusion | 174-177°C (décomposition) |
| Rendement | 55 % |
| Solvant de recristallisation | Ethanol absolu |

### PREPARATION 2 : 6-(3-CHLOROPROPYL)BENZOTHIAZOLINONE

### Réactifs :

| | |
|---|---|
| 6-(3-chloropropionyl)benzothiazolinone | 0,10 mole (24,16 g) |
| Acide trifluoroacétique | 0,80 mole (61,60 cm³) |
| Triéthylsilane | 0,20 mole (32,00 cm³) |

### Mode opératoire :

Dans une fiole à col rodé de 250 cm³ dissoudre 0,1 mole de 6-(3-chloropropionyl)benzothiazolinone dans 0,8 mole d'acide trifluoroacétique. A l'aide d'une ampoule à brome, introduire goutte à goutte et sous agitation magnétique 0,2 mole de triéthylsilane.

Adapter une garde de chlorure de calcium et maintenir l'agitation à température ambiante pendant le temps nécessaire.

Verser le mélange réactionnel dans 500 cm³ d'eau glacée.

Essorer le précipité obtenu, le laver à l'eau jusqu'à neutralité des eaux de lavage, le sécher puis le recristalliser.

| | |
|---|---|
| Temps de réaction | 50 heures |
| Masse moléculaire | 227,71 g.mole⁻¹ pour C₁₀H₁₀ClNOS |
| Point de fusion | 131-133°C |
| Rendement | 86 % |
| Solvant de recristallisation | Toluène |

### PREPARATION 3 : 3-METHYL 6-(3-CHLOROPROPIONYL)BENZOTHIAZOLINONE

### Réactifs :

| | |
|---|---|
| 3-Méthylbenzothiazolinone | 0,05 mole (8,25 g) |
| Chlorure de 3-chloropropionyle | 0,06 mole (5,72 cm³) |
| Chlorure d'aluminium | 0,40 mole (52,50 g) |
| Diméthylformamide | 0,20 mole (9,20 cm³) |

### Mode opératoire :

II est identique à celui utilisé pour l'obtention de la 6-(3-chloropropionyl)benzothiazolinone en remplaçant la benzothiazolinone par la 3-méthyl benzothiazolinone.

| | |
|---|---|
| Masse moléculaire | 237,27 g.mole⁻¹ pour C₁₁H₁₀ClNO₂S |
| Point de fusion | 174-177°C |
| Rendement | 60 % |
| Solvant de recristallisation | Ethanol absolu |

### PREPARATION 4 : 3-METHYL 6-(3-CHLOROPROPYL)BENZOTHIAZOLINONE

### Réactifs :

| | |
|---|---|
| 3-Méthyl 6-(3-chloropropionyl)benzothiazolinone | 0,1 mole (23,72 g) |
| Acide trifluoroacétique | 0,8 mole (61,60 cm³) |
| Triéthylsilane | 0,2 mole (32,00 cm³) |

### Mode opératoire :

II est identique à celui utilisé pour l'obtention de la 6-(3-chloropropyl)benzothiazolinone en remplaçant la 6-(3-chloropropionyl) benzothiazolinone par la 3-méthyl 6-(3-chloropropionyl) benzothiazolinone.

| | |
|---|---|
| Temps de réaction | 72 heures |
| Masse moléculaire | 241,73 g.mole⁻¹ pour C₁₁H₁₂ClNOS |
| Point de fusion | 41-43°C |
| Rendement | 84 % |
| Solvant de recristallisation | Cyclohexane |

### EXEMPLE 1 : 6-(3-MORPHOLINOPROPYL)BENZOTHIAZOLINONE

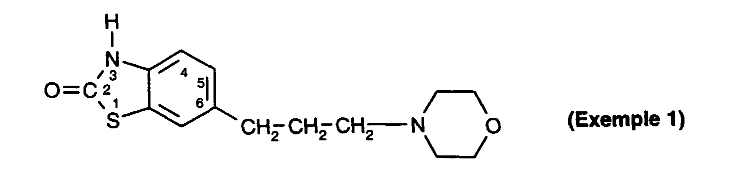

Dans une fiole à col rodé de 250 cm³ munie d'un réfrigérant et contenant 80 cm³ d'alcool absolu, introduire 0,02 mole (4,55 g) de 6-(3-chloropropyl)benzothiazolinone, 0,03 mole (2,60 cm³) de morpholine et 0,03 mole (4,20 cm³) de triéthylamine.

Chauffer le mélange à reflux pendant trente heures. Essorer le précipité de chlorhydrate de triéthylamine puis évaporer le filtrat au bain marie sous pression réduite. Reprendre le résidu par une solution aqueuse d'acide chlorhydrique 1N et extraire avec de l'éther.

La phase aqueuse est alcalinisée avec une solution de soude à 10 %. Extraire deux fois à l'éther, réunir les phases éthérées, les sécher sur du chlorure de calcium, filtrer et évaporer sous pression réduite.

Reprendre le résidu par de l'éther anhydre, faire barboter de l'acide chlorhydrique gazeux, essorer le précipité formé et le recristalliser. On obtient le composé du titre sous forme de chlorhydrate.
Masse moléculaire : 314,83 g.mol⁻¹ pour C₁₄H₁₉ClN₂O₂S
Rendement : 62 %
Point de fusion : 239-241°C
Solvant de recristallisation : Alcool absolu

| **Analyse élémentaire :** | | | |
|---|---|---|---|
| | % C | % H | % N |
| Calculée | 54,40 | 6,08 | 8,89 |
| Trouvée | 54,33 | 5,86 | 8,72 |

### Spectrométrie dans l'infra-rouge :

| | |
|---|---|
| 2920 cm⁻¹ | ν CH (alkyles) |
| 2660-2450 cm⁻¹ | ν NH⁺ (chlorhydrate) |
| 1690 cm⁻¹ | ν CO (NCOS) |
| 1600 cm⁻¹ | ν C=C (aromatiques) |

### Spectrométrie de résonance magnétique nucléaire (80MHz ; DMSO,d₆) :

| | | |
|---|---|---|
| δ = 2,20 | ppm (massif, 2H) | CH₂-CH₂-CH₂ |
| δ = 2,70 | ppm (triplet, 2H) | Benzothiazolinone-CH₂ |
| δ = 7,00 | ppm (singulet, 2H) | H_{4,5} (benzothiazolinone) |
| δ = 7,40 | ppm (singulet, 1H) | H₇ (benzothiazolinone) |
| δ = 11,80 | ppm (signal, 2H) | NH, NH⁺échangeables dans D₂O |

### EXEMPLE 2 : 3-METHYL 6-(3-MORPHOLINOPROPYL)BENZOTHIAZOLINONE

### Réactifs :

| | |
|---|---|
| 3-méthyl 6-(3-chloropropyl)benzothiazolinone | 0,02 mole (4,83 g) |
| Morpholine | 0,03 mole (2,60 cm³) |
| Triéthylamine | 0,03 mole (4,20 cm³) |
| Acétone anhydre | 60 cm³ |

### Mode opératoire :

Il est identique à celui utilisé dans l'exemple 1 en remplaçant la 6-(3-chloropropyl)benzothiazolinone par la 3-méthyl 6-(3-chloropropyl)benzothiazolinone.

| | |
|---|---|
| Masse moléculaire | 328,86 g.mole⁻¹ pour C₁₅H₂₁ClN₂O₂S |
| Rendement | 67 % |
| Point de fusion (chlorhydrate) | 210-212°C |
| Solvant de recristallisation | Alcool absolu |

| **Analyse élémentaire :** | | | |
|---|---|---|---|
| | % C | % H | % N |
| Calculée | 54,78 | 6,43 | 8,52 |
| Trouvée | 54,76 | 6,70 | 8,70 |

### Spectrométrie dans l'infra-rouge :

| | |
|---|---|
| 2900 cm⁻¹ | ν CH (alkyles) |
| 2660-2450 cm⁻¹ | ν NH⁺(chlorhydrate) |
| 1670 cm⁻¹ | ν CO (NCOS) |
| 1600 cm⁻¹ | ν C=C (aromatiques) |

### Spectrométrie de résonance magnétique nucléaire (80MHz ; DMSO,d₆) :

| | | |
|---|---|---|
| δ = 2,10 | ppm (multiplet, 2H) | CH₂-CH₂-CH₂ |
| δ = 2,75 | ppm (triplet, 2H) | Benzothiazolinone-CH₂ |
| δ = 3,40 | ppm (singulet, 3H) | NCH₃ |
| δ = 3,90 | ppm (multiplet, 4H) | CH₂-O-CH₂ |
| δ = 7,35 | ppm (multiplet, 3H) | H_{4,5,7} (benzothiazolinone) |
| δ = 11,20 | ppm (signal, 1H) | NH⁺ échangeable dans D₂O |

### EXEMPLE 3 : 6-[3-(3,5-DIMETHYL)MORPHOLINOPROPYL]BENZOTHIAZOLINONE

En procédant de la même façon que dans l'exemple 1, mais en remplaçant la morpholine par la 3,5-diméthylmorpholine, on obtient le composé du titre.

### EXEMPLE 4 : 3-METHYL 6-[3-(3,5-DIMETHYL)MORPHOLINOPROPYL]BENZOTHIAZOLINONE

En procédant de la même façon que dans l'exemple 2, mais en remplaçant la morpholine par la 3,5-diméthylmorpholine, on obtient le composé du titre.

### EXEMPLE 5 : 6-(3-PIPERIDIN-1-YL-PROPYL)BENZOTHIAZOLINONE

En procédant de la même façon que dans l'exemple 1, mais en remplaçant la morpholine par la pipéridine, on obtient le composé du titre.

### EXEMPLE 6 : 3-METHYL 6-(3-PIPERIDIN-1-YL-PROPYL)BENZOTHIAZOLINONE

En procédant de la même façon que dans l'exemple 2, mais en remplaçant la morpholine par la pipéridine, on obtient le composé du titre.

### EXEMPLE 7 : 6-(3-PYRROLIDIN-1-YL-PROPYL)BENZOTHIAZOLINONE

En procédant de la même façon que dans l'exemple 1, mais en remplaçant la morpholine par la pyrrolidine, on obtient le composé du titre.

### EXEMPLE 8 : 3-METHYL 6-(3-PYRROLIDIN-1-YL-PROPYL)BENZOTHIAZOLINONE

En procédant de la même façon que dans l'exemple 2, mais en remplaçant la morpholine par la pyrrolidine, on obtient le composé du titre.

### EXEMPLE 9 : 6-(3-N,N-DIPROPYLAMINOPROPYL)BENZOTHIAZOLINONE

En procédant de la même façon que dans l'exemple 1, mais en remplaçant la morpholine par la N,N-dipropylamine, on obtient le produit du titre.

### EXEMPLE 10 : 3-METHYL 6-(3-N,N-DIPROPYLAMINOPROPYL)BENZOTHIAZOLINONE

En procédant de la même façon que dans l'exemple 2, mais en remplaçant la morpholine par la N,N-dipropylamine, on obtient le produit du titre.

### EXEMPLE 11 : 6-(3-N-CYCLOHEXYLAMINOPROPYL)BENZOTHIAZOLINONE

En procédant de la même façon que dans l'exemple 1, mais en remplaçant la morpholine par la cyclohexylamine, on obtient le produit du titre.

### EXEMPLE 12 : 3-METHYL 6-(3-N-CYCLOHEXYLAMINOPROPYL)BENZOTHIAZOLINONE

En procédant de la même façon que dans l'exemple 2, mais en remplaçant la morpholine par la cyclohexylamine, on obtient le produit du titre.

### EXEMPLE 13 : 6-(3-BENZYLAMINOPROPYL)BENZOTHIAZOLINONE

En procédant de la même façon que dans l'exemple 1, mais en remplaçant la morpholine par la benzylamine, on obtient le produit du titre.

### EXEMPLE 14 : 3-METHYL 6-(3-BENZYLAMINOPROPYL)BENZOTHIAZOLINONE

En procédant de la même façon que dans l'exemple 2, mais en remplaçant la morpholine par la benzylamine, on obtient le produit du titre.

### EXEMPLE 15 : 6-[3-(4-METHOXYBENZYL)AMINOPROPYL)BENZOTHIAZOLINONE

En procédant de la même façon que dans l'exemple 1, mais en remplaçant la morpholine par la 4-méthoxybenzylamine, on obtient le produit du titre.

### EXEMPLE 16 : 3-METHYL 6-[3-(4-METHOXYBENZYL)AMINOPROPYL)BENZOTHIAZOLINONE

En procédant de la même façon que dans l'exemple 2, mais en remplaçant la morpholine par la 4-méthoxybenzylamine, on obtient le produit du titre.

### EXEMPLE 17 : 6-{3-[1-(NAPHT-1-YL)ETHYLAMINO]PROPYL)BENZOTHIAZOLINONE

En procédant de la même façon que dans l'exemple 1, mais en remplaçant la morpholine par la 1-(1-naphtyl)éthylamine, on obtient le produit du titre.

### EXEMPLE 18 : 3-METHYL 6-{3-[1-(NAPHT-1-YL)ETHYLAMINO]PROPYL}BENZOTHIAZOLINONE

En procédant de la même façon que dans l'exemple 2, mais en remplaçant la morpholine par la 1-(1-naphtyl)éthylamine, on obtient le produit du titre.

### EXEMPLE 19 : 6-[3-(4-METHYLBENZYL)AMINOPROPYL]BENZOTHIAZOLINONE

En procédant de la même façon que dans l'exemple 1, mais en remplaçant la morpholine par la 4-méthylbenzylamine, on obtient le produit du titre.

### EXEMPLE 20 : 3-METHYL 6-[3-(4-METHYLBENZYL)AMINOPROPYL]BENZOTHIAZOLINONE

En procédant de la même façon que dans l'exemple 2, mais en remplaçant la morpholine par la 4-méthylbenzylamine, on obtient le produit du titre.

### EXEMPLE 21 : 6-[3-(4-TRIFLUOROMETHYLBENZYL)AMINOPROPYL]BENZOTHIAZOLINONE

En procédant de la même façon que dans l'exemple 1, mais en remplaçant la morpholine par la 4-trifluorométhylbenzylamine, on obtient le produit du titre.

### EXEMPLE 22 : 3-METHYL 6-[3-(4-TRIFLUOROMETHYLBENZYL)AMINOPROPYL] BENZOTHIAZOLINONE

En procédant de la même façon que dans l'exemple 2, mais en remplaçant la morpholine par la 4-trifluorométhylbenzylamine, on obtient le produit du titre.

### EXEMPLE 23 : 6-[3-(3,4-DICHLOROBENZYL)AMINOPROPYL]BENZOTHIAZOLINONE

En procédant de la même façon que dans l'exemple 1, mais en remplaçant la morpholine par la 3,4-dichlorobenzylamine, on obtient le produit du titre.

### EXEMPLE 24 : 3-METHYL 6-[3-(3,4-DICHLOROBENZYL)AMINOPROPYL]BENZOTHIAZOLINONE

En procédant de la même façon que dans l'exemple 2, mais en remplaçant la morpholine par la 3,4-dichlorobenzylamine, on obtient le produit du titre.

### EXEMPLE 25 : 6-(3-THIOMORPHOLINOPROPYL)BENZOTHIAZOLINONE

En procédant de la même façon que dans l'exemple 1, mais en remplaçant la morpholine par la thiomorpholine, on obtient le produit du titre.

### EXEMPLE 26 : 3-METHYL 6-(3-THIOMORPHOLINOPROPYL)BENZOTHIAZOLINONE

En procédant de la même façon que dans l'exemple 2, mais en remplaçant la morpholine par la thiomorpholine, on obtient le produit du titre.

### EXEMPLE 27 : 3-METHYL 6-(3-ALLYLAMINOPROPYL)BENZOTHIAZOLINONE

En procédant de la même façon que dans l'exemple 2, mais en remplaçant la morpholine par l'allylamine, on obtient le produit du titre.

### EXEMPLE 28 : 3-METHYL-6-[2-(4-BENZYLPIPERAZIN-1-YL)ETHYL] BENZOTHIAZOLINONE

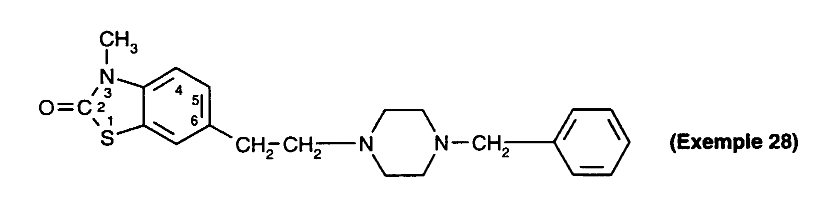

| **Réactifs** | |
|---|---|
| 3-méthyl 6-(2-bromoéthyl)benzothiazolinone | 0,0074 mole (2 g) |
| 4-benzylpipérazine | 0,0074 mole (1,30 cm³) |
| Carbonate de potassium | 0,029 mole (4 g) |
| Diméthylformamide anhydre | 50 cm³ |

### Mode opératoire :

Dans une fiole à col rodé de 250 cm³ munie d'un réfrigérant et contenant 30 cm³ de diméthylformamide anhydre, introduire 0,0074 mole de 4-benzylpipérazine et 0,029 mole de carbonate de potassium. Chauffer à reflux pendant 30 minutes, ensuite additionner 0,0074 mole de 3-méthyl-6-(2-bromoéthyl)benzothiazolinone en solution dans 20 cm³ de diméthylformamide.

Chauffer le mélange réactionnel pendant 12 heures. Laisser refroidir et essorer le précipité minéral. Verser le filtrat dans 30 cm³ d'eau distillée et acidifier. Laisser agiter pendant 30 minutes. Extraire à l'acétate d'éthyle pour éliminer la matière première résiduelle. La phase aqueuse est aussitôt alcalinisée.

Extraire la phase organique à l"acétate d'éthyle. Réunir les fractions organiques et sécher l'ensemble, filtrer puis évaporer au bain marie sous pression réduite.

Reprendre le résidu par 50 cm³ d'alcool absolu, faire barboter de l'acide chlorhydrique gazeux, essorer le précipité formé et le recristalliser. On obtient le composé du titre sous forme de dichlorhydrate.

| | |
|---|---|
| Masse moléculaire | 440,36 g.mole⁻¹ pour C₂₁ H₂₇Cl₂N₃OS |
| Rendement | 45 % |
| Point de fusion | > 270°C |
| Solvant de recristallisation | Alcool absolu |

| **Analyse élémentaire :** | | | |
|---|---|---|---|
| | % C | % H | % N |
| Calculée | 57,27 | 6,18 | 9,54 |
| Trouvée | 57,31 | 6,15 | 9,50 |

### Spectrométrie dans l'infra-rouge :

| | |
|---|---|
| 2900 cm⁻¹ | ν CH(alkyles) |
| 2340 cm⁻¹ | ν NH⁺ (chlorhydrate) |
| 1690 cm⁻¹ | ν CO (NCOS) |
| 1600 cm⁻¹ | ν C=C (aromatiques) |

### Spectrométrie de résonance magnétique nucléaire (80MHz ; DMSO,d₆) :

| | | |
|---|---|---|
| δ = 2,80-3,14 | ppm (massif, 12H) | éthyle et pipérazine |
| δ = 3,40 | ppm (singulet, 3H) | NCH₃ |
| δ = 4,10 | ppm (singulet, 2H) | CH₂-C₆H₅ |
| δ = 7,20-7,70 | ppm (massif, 8H) | H aromatiques |
| δ = 11,00 | ppm (signal, 2H) | 2NH⁺échangeables dans D₂O |

### EXEMPLE 29 : 3-METHYL-6-[4-(4-BENZYLPIPERAZIN-1-YL) BUTYL]BENZOTHIAZOLINONE

### Réactifs :

| | |
|---|---|
| 3-méthyl-6-(4-bromobutyl)benzothiazolinone | 0,01 mole (3 g) |
| 4-benzylpipérazine | 0,01 mole (1,80 cm³) |
| carbonate de potassium | 0,04 mole (5,5 g) |
| Diméthylformamide anhydre | 45 cm³ |

### Mode opératoire :

II est identique à celui utilisé dans l'exemple 28 en remplaçant la 3-méthyl 6-(2-bromoéthyl)benzothiazolinone par la 3-méthyl 6-(4-bromobutyl)benzothiazolinone.

### Données physicochimiques du dichlorhydrate :

| | |
|---|---|
| Masse moléculaire | 468,42 g.mole⁻¹ pour C₂₃H₃₁Cl₂N₃OS |
| Rendement | 55 % |
| Point de fusion | > 250°C |
| Solvant de recristallisation | Alcool absolu |

| **Analyse élémentaire :** | | | |
|---|---|---|---|
| | % C | % H | % N |
| Calculée | 58,97 | 6,67 | 8,97 |
| Trouvée | 58,59 | 6,72 | 8,96 |

### Spectrométrie dans l'infra-rouge :

| | |
|---|---|
| 3400-2320 cm⁻¹ | ν NH⁺(chlorhydrate) |
| 3040-2840 cm⁻¹ | ν CH (alkyles) |
| 1670 cm⁻¹ | ν CO (NCOS) |
| 1600 cm⁻¹ | ν C=C (aromatiques) |

### Spectrométrie de résonance magnétique nucléaire (80MHz ; DMSO,d₆) :

| | | |
|---|---|---|
| δ = 1,70 | ppm (massif, 4H) | CH₂-CH₂-CH₂-CH₂ |
| δ = 2,00-2,75 | ppm (massif, 4H) | CH₂-CH₂-CH₂-CH₂ |
| δ = 3,20-3,70 | ppm (massif, 11H) | NCH₃ et pipérazine |
| δ = 6,90-7,40 | ppm (massif, 8H) | H aromatiques |

### EXEMPLE 30 : 3-METHYL-6-{2-[4-(2,4-DICHLOROBENZYL)PIPERAZIN-1-YL) ETHYL]BENZOTHIAZOLINONE

### Réactifs :

| | |
|---|---|
| 3-méthyl 6-(2-bromoéthyl)benzothiazolinone | 0,0073 mole (2 g) |
| Dichlorhydrate de 2,4-dichlorobenzylpipérazine | 0,0073 mole (2,3 g) |
| Triéthylamine | 0,0150 mole (2 cm³) |
| Acétone anhydre | 70 cm³ |

### Mode opératoire :

II est identique à celui utilisé dans l'exemple 28 en remplaçant la 1-benzylpipérazine par la 1-(2,4-dichlorobenzyl)pipérazine.

### Données physicochimiques du dichlorhydrate :

| | |
|---|---|
| Masse moléculaire | 509,26 g.mole⁻¹ pour C₂₁H₂₅Cl₄N₃OS |
| Rendement | 60 % |
| Point de fusion | 189-191°C |
| Solvant de recristallisation | Alcool absolu |

| **Analyse élémentaire :** | | | |
|---|---|---|---|
| | %C | %H | %N |
| Calculée | 49,52 | 4,94 | 8,25 |
| Trouvée | 49,37 | 5,05 | 8,27 |

### Spectrométrie dans l'infra-rouge :

| | |
|---|---|
| 2940 cm⁻¹ | ν CH (alkyles) |
| 2620-2180 cm⁻¹ | ν NH⁺(chlorhydrate) |
| 1700 cm⁻¹ | ν CO (NCOS) |
| 1580 cm⁻¹ | ν C=C (aromatiques) |

### Spectrométrie de résonance magnétique nucléaire (300MHz ; DMSO,d₆) :

| | | |
|---|---|---|
| δ = 3,60 | ppm (multiplet, 2H) | CH₂-C₆ H₃-Cl₂ |
| δ = 3,70 | ppm (singulet, 3H) | NCH₃ |
| δ = 7,20-7,70 | ppm (massif, 6H) | H aromatiques |
| δ = 8,70 | ppm (signal, 1 H) | NH⁺échangeables dans D₂O |
| δ = 10,85 | ppm (signal, 1 H) | NH⁺échangeables dans D₂O |

### EXEMPLE 31 : 3-METHYL-6-{4-[4-(2,4-DICHLOROBENZYL)PIPERAZIN-1-YL) BUTYL]BENZOTHIAZOLINONE

### Réactifs :

| | |
|---|---|
| 3-méthyl 6-(4-bromobutyl)benzothiazolinone | 0,0066 mole (2 g) |
| Dichlorhydrate de 2,4-dichlorobenzyl- | |
| pipérazine | 0,0066 mole (2,1 g) |
| Triéthylamine | 0,0132 mole (1,80 cm³) |
| Acétone anhydre | 50 cm³ |

### Mode opératoire :

II est identique à celui utilisé dans l'exemple 29 en remplaçant la 1-benzylpipérazine par la 1-(2,4-dichlorobenzyl)pipérazine

### Données physicochimiques (dichlorhydrate) :

| | |
|---|---|
| Masse moléculaire | 537,31 g.mole⁻¹ pour C₂₃H₂₉Cl₄N₃OS |
| Rendement | 42 % |
| Point de fusion | 257-259 °C |
| Solvant de recristallisation : | Alcool absolu |

| **Analyse élémentaire :** | | | |
|---|---|---|---|
| | %C | %H | %N |
| Calculée | 51,41 | 5,44 | 7,82 |
| Trouvée | 51,27 | 5,36 | 7,78 |

### Spectrométrie dans l'infra-rouge :

| | |
|---|---|
| 3400-2360 cm⁻¹ | ν NH⁺(chlorhydrate) |
| 3040-2840 cm⁻¹ | ν CH (alkyles) |
| 1680 cm⁻¹ | ν CO (NCOS) |
| 1580 cm⁻¹ | ν C=C (aromatiques) |

### Spectrométrie de résonance magnétique nucléaire (80MHz ; DMSO,d₆) :

| | | |
|---|---|---|
| δ = 4,25 | ppm (singulet, 2H) | CH₂-C₆ H₃-Cl₂ |
| δ = 7,00-8,00 | ppm (massif, 6H) | H aromatiques |
| δ = 11,50 | ppm (signal, 2H) | 2NH⁺échangeables dans D₂O |

### EXEMPLE 32 : 3-METHYL-6-{2-[4-(3,4-DICHLOROBENZYL)PIPERAZIN-1-YL) ETHYL]BENZOTHIAZOLINONE

### Réactifs :

| | |
|---|---|
| 3-méthyl 6-(2-bromoéthyl)benzothiazolinone | 0,0073 mole (2 g) |
| Dichlorhydrate de 3,4-dichlorobenzyl- | |
| pipérazine | 0,0073 mole (2,1 g) |
| Carbonate de potassium | 0,029 mole (4 g) |
| Acétone anhydre | 50 cm³ |

### Mode opératoire :

II est identique à celui utilisé dans l'exemple 29 en remplaçant la 1-benzylpipérazine par la 1-(3,4-dichlorobenzyl)pipérazine.

### Données physicochimiques du dichlorhydrate :

| | |
|---|---|
| Masse moléculaire | 509,26 g.mole⁻¹ pour C₂₁H₂₅Cl₄N₃OS |
| Rendement | 56 % |
| Point de fusion | > 250 °C |
| Solvant de recristallisation | Alcool absolu |

| **Analyse élémentaire :** | | | |
|---|---|---|---|
| | % C | % H | % N |
| Calculée | 49,52 | 4,94 | 8,25 |
| Trouvée | 49,37 | 5,05 | 8,27 |

### Spectrométrie dans l'infra-rouge :

| | |
|---|---|
| 2900 cm⁻¹ | ν CH (alkyles) |
| 2300 cm⁻¹ | ν NH⁺(chlorhydrate) |
| 1700 cm⁻¹ | ν CO (NCOS) |
| 1590-1570 cm⁻¹ | ν C=C (aromatiques) |

### Spectrométrie de résonance magnétique nucléaire (80MHz ; DMSO,d₆) :

| | | |
|---|---|---|
| δ = 2,80-3,15 | ppm (massif, 12H) | éthylpipérazine |
| δ = 3,40 | ppm (singulet, 3H) | NCH₃ |
| δ = 4,10 | ppm (singulet, 2H) | CH₂-C₆ H₃-Cl₂ |
| δ = 7,20-7,70 | ppm (massif, 6H) | H aromatiques |
| δ = 11,00 | ppm (signal, 2H) | 2NH⁺échangeables dans D₂O |

### EXEMPLE 33 : 3-METHYL-6-{4-[4-(3,4-DICHLOROBENZYL)PIPERAZIN-1YL) BUTYL]BENZOTHIAZOLINONE

### Réactifs :

| | |
|---|---|
| 3-méthyl 6-(4-bromobutyl)benzothiazolinone | 0,0033 mole (1 g) |
| Dichlorhydrate de 3,4-dichlorobenzylpipérazine | 0,0033 mole (1 g) |
| Carbonate de potassium | 0,0132 mole (1,8 g) |
| Acétone anhydre | 40 cm³ |

### Mode opératoire :

Il est identique à celui utilisé dans l'exemple 29 en remplaçant la 1-benzylpipérazine par la 1-(3,4-dichlorobenzyl)pipérazine.

### Données physicochimiques du dichlorhydrate :

| | |
|---|---|
| Masse moléculaire | 537,31 g.mole⁻¹ pour C₂₃H₂₉Cl₄N₃OS |
| Rendement | 45 % |
| Point de fusion (chlorhydrate) | > 250 °C |
| Solvant de recristallisation | Alcool absolu |

| **Analyse élémentaire :** | | | |
|---|---|---|---|
| | % C | % H | % N |
| Calculée | 51,41 | 5,44 | 7,82 |
| Trouvée | 51,30 | 5,43 | 7,75 |

### Spectrométrie dans l'infra-rouge :

| | |
|---|---|
| 3400-2360 cm⁻¹ | ν NH⁺(chlorhydrate) |
| 3040-2960 cm⁻¹ | ν CH (alkyles) |
| 1670 cm⁻¹ | ν CO (NCOS) |
| 1600 cm⁻¹ | ν C=C (aromatiques) |

### Spectrométrie de résonance magnétique nucléaire (80MHz ; DMSO,d₆) :

| | | |
|---|---|---|
| δ = 1,40-1,80 | ppm (massif, 4H) | CH₂-CH₂-CH₂-CH₂ |
| δ = 4,20 | ppm (singulet, 2H) | CH₂-C₆ H₃-Cl₂ |
| δ = 7,00-8,00 | ppm (massif, 6H) | H aromatiques |
| δ = 11,25 | ppm (signal, 2H) | 2NH⁺échangeables dans D₂O |

### EXEMPLE 34 : 6-{3-[4-(2-METHOXYPHENYL)PIPERAZINO]PROPYL}BENZOTHIAZOLINONE

Point de fusion (chlorhydrate) : 224-225°C

### EXEMPLE 35 : 3-METHYL-6-{3-[4-(2-METHOXYPHENYL)PIPERAZINO]PROPYL}BENZOTHIAZOLINONE

Point de fusion (chlorhydrate) : 205-207°C

### ETUDE PHARMACOLOGIQUE DES DERIVES DE L'INVENTION

### EXEMPLE A : ETUDE DE LA TOXICITE AIGUE

La toxicité aiguë a été appréciée après administration orale à des lots de 8 souris (26 ± 2 grammes) d'une dose de 100 mg.kg⁻¹ des composés de l'invention. Les animaux ont été observés à intervalles réguliers au cours de la première journée et quotidiennement pendant les deux semaines suivant le traitement.
Il apparaît que les composés de l'invention sont totalement atoxiques. Ils n'entrainent aucun décès après administration à une dose de 100 mg.kg⁻¹ et on ne constate pas de troubles après administration de cette dose.

### EXEMPLE B : BILAN RECEPTORIEL D'AFFINITE IN VITRO

Les produits sont testés sur chaque récepteur à 5 concentrations différentes (10⁻⁵M, 10⁻⁶M, 10⁻⁷M, 10⁻⁸M, 10⁻⁹M) en triplicata. Lorsque le coefficient de liaison IC₅₀ est inférieur à une concentration de 10⁻⁶M, le Ki est mesuré en utilisant 12 concentrations du produit.

Le tableau ci-dessous regroupe les récepteurs pour lesquels l'affinité des composés de l'invention à été déterminée, le tissu choisi, la concentration retenue pour déterminer la liaison non spécifique et le radioligand utilisé pour marquer le récepteur.

| **Récepteur ou site** | **Radioligand** | **Liaison non spécifique** | **Tissu** |
|---|---|---|---|
| 5-HT_{1A} | [³H] 8-OH DPAT | 10⁻⁵M Buspirone | Hippocampe de boeuf + cortex frontal |
| 5-HT_{1B} | [³H] Cyanopindolol ou 5-OH tryptamine ou propanolol | 10⁻⁵M Sérotonine froide | Cerveau de rat + cortex frontal + striatum |
| 5-HT_{1C} | [³H] N-méthyl mésulergine | 10⁻⁵M Miansérine | Plexus choroïde de porc |
| 5-HT₂ | [³H] kétansérine | 10⁻⁵M Spipérone | Cortex frontal de boeuf |
| 5-HT₃ | [³H] Quipazine ou BRL 93694 | 10⁻⁵M ICS 255930 | cellules NG 108-15 |
| α₁ | [³H] Prazosin | 10⁻⁵M Phentolamine | Cerveau de rat |
| α₂ | [³H] Rauwolscine | 10⁻⁵M Yohimbine | Cerveau de rat |
| D₁ | [³H] SCH 23390 | 10⁻⁶M Butaclamol | Striatum de boeuf |
| D₂ | [³H] Raclopride | 10⁻⁶_{M} spipérone ou 10⁻⁶M Halopéridol | Striatum de boeuf |
| M1 | [³H] Télenzépine | 10⁻⁵M Atropine | Cortex de rat |
| H₁ | [³H] Pyrilamine | 10⁻⁶M Chlorphéniramine | Cortex de veau |
| σ | [³H] DTG | 10⁻⁶M 3-PPP | Hippocampe de veau |

Les résultats du test ont montré que les composés de l'invention sont de puissants et sélectifs ligands des récepteurs σ. A titre de comparaison les dérivés de la présente invention ont une affinité 100 à 1 000 fois plus puissante que les dérivés de la demande EP 478 446 pour le récepteur sigma, et plus particulièrement les exemples 28 à 32 et 172 de cette demande structuralement les plus proches de ceux de l'invention présente.

Par ailleurs, les dérivés de la présente invention sont également beaucoup plus sélectifs que les dérivés de la demande EP 478 446. En particulier, ils sont 100 à 1 000 fois moins affins pour les récepteurs D₂ que les dérivés de la demande EP 478 446.

### EXEMPLE C : ANTAGONISME DE l'HYPERMOTILITE INDUITE PAR L'AMPHETAMINE

On injecte à des groupes de 10 souris NMRI-CERJ par voie intrapéritonéale (IP) 4 mg kg⁻¹ de d-amphétamine juste après l'injection IP du composé à tester, et on place les souris dans un actimètre pendant 30 minutes.
Le nombre d'interruptions des cellules photoélectriques est compté, de même que le comportement stéréotypé.
L'activité des composés testés est exprimée en pourcentage de l'antagonisme de l'hyperactivité induite par l'amphétamine. Les composés de l'invention sont de puissants antagonistes de l'hypermotilité induite par l'amphétamine ce qui permet de conclure à une activité dans les troubles du système nerveux central pour les produits de l'invention.

### EXEMPLE D : RECHERCHE D'EFFET CATALEPSIGENE

L'effet catalepsigène est recherché chez le rat par voie intrapéritonéale. Ce test est prédictif de l'existence d'effets secondaires du type extrapyramidaux. 6 groupes de rats Wistar ont reçu une injection des composés de l'invention et sont ensuite testés pour leur activité catalepsigène à 30 minutes d'intervalle. L'halopéridol est utilisé comme référence.

Les résultats montrent que les composés de l'invention ont un très faible pouvoir catalepsigène comparé à l'halopéridol qui produit à la dose de 2 mg.kg⁻¹ un effet catalepsigène de 95 % dans les mêmes conditions d'études. Ce résultat confirme l'absence d'effets secondaires de type extrapyramidaux des produits de l'invention qui pouvait être attendu suite aux résultats de liaisons réceptorielles (exemple B).

### EXEMPLE E : ETUDE DE L'ACTIVITE ANTIDEPRESSIVE DES COMPOSES DE L'INVENTION

### PRINCIPE :

L'étude des produits est réalisée sur le modèle du "renoncement appris" qui consiste à induire chez l'animal, par une série d'évènements aversifs incontrôlables, un déficit lors des tâches d'évitement ultérieures.

### PROTOCOLE :

Ce test a été mis au point par Sherman A.D., Sacquitne J.L., et Petty F. (Pharmacol. Biochem. Behav., 1982, 16, 449-454). Nous utilisons des rats mâles Wistar d'un poids compris entre 180 et 200 grammes. Les animaux sont maintenus à l'animalerie une semaine avant le test, dans des boîtes en plastique, par groupes de 10, à une température ambiante de 21° C ± 1° C, avec libre accès à l'eau et à la nourriture.
Les animaux sont ensuite isolés dans des boîtes de petites dimensions et sont soumis à 60 chocs électriques inévitables (0,8 mA toutes les minutes ± 15 secondes). Un groupe de rats témoins ne reçoit pas de chocs électriques.
La capacité des animaux à réaliser un apprentissage d'évitement (passage d'un compartiment à l'autre, afin d'éviter les chocs électriques) est appréciée 48 heures après et pendant 3 jours consécutifs. Lors des séances d'apprentissage, les animaux subissent 2 essais par minute pendant 15 minutes. Le nombre d'échecs d'échappement est noté pour chaque rat. Les animaux sont traités (i.p. : 0,5 cm³/100 g) à jeun 6 heures après les chocs inévitables et 4 jours de suite, le matin 30 minutes avant la séance d'apprentissage et le soir entre 18 et 19 h.
Les produits étudiés sont mis en solution dans de l'eau distillée.
Les produits étudiés sont administrés aux doses 0,05 mg.kg⁻¹/jour.

### RESULTATS :

Le test prouve que les produits de l'invention diminuent significativement le nombre d'échecs d'échappement ce qui traduit, pour certains produits de l'invention, une forte activité de type antidépressive.

### EXEMPLE F : ETUDE DE L'ACTIVITE ANXIOLYTIQUE - TEST DIT DES CAGES CLAIRE/OBSCURE CHEZ LA SOURIS

### PRINCIPE :

Nous nous proposons d'étudier les effets anxiolytiques des composés de l'invention, au moyen du test des cages claires/obscures chez la souris.

### PROTOCOLE :

Ce test a été mis au point par Crawley et al. (1981, Pharmacol. Biochem. Behav.), puis modifié et comportementalement validé.

Il s'agit de deux cages de tailles égale (20 X 20 X 14 cm) en PVC. L'une est fortement éclairée par une lampe de 100 W(lumière "froide"), l'autre est obscurcie. Les deux cages sont séparées l'une de l'autre au moyen d'un petit tunnel opaque (5 X 7 cm). Les souris sont individuellement introduites dans la cage obscure, et on enregistre, au moyen de claviers reliés à un ordinateur, durant 5 minutes, le temps passé par les animaux dans la cage éclairée ainsi que le nombre des transitions entre la cage obscure et la cage éclairée.

Chaque groupe expérimental comprend au minimum 15 animaux.

### RESULTATS :

L'administration, par voie intrapéritonéale de certains produits de l'invention entraine une augmentation du temps passé par les souris dans la cage éclairée et du nombre des transitions entre la cage obscure et la cage éclairée.

Cette augmentation significative des deux paramètres étudiés montrent la remarquable activité anxiolytique de certains composés de l'invention.

### EXEMPLE G : RECHERCHE D'UNE ACTIVITE DE TYPE ANTIARTHRITIQUE CHEZ LE RAT

On utilise des groupes de 5 rats Lewis mâle ou femelle d'un poids de 130 à 150 g. Une suspension de 0,3 mg de Mycobactérium tuberculorsis tués dans 0,1 cm³ d'huile minérale (Adjuvant de Freund complet, CFA) est administré dans la région subplantaire de la patte arrière droite le Jour 1. Les volumes des pattes arrières sont mesurés par déplacement d'eau les Jours 0, 1, 5, 14 et 18. Les rats sont pesés aux jours 0 et 18. Les prdduits à tester sont placés en suspension dans de la carboxyméthyl cellulose et administrés oralement pendant 5 jours consécutifs des jours 1 à 5.
Parallèlement un groupe témoin est utilisé afin d'éliminer les artefacts résultant de la manipulation des animaux. Un groupe traité par un produit de référence permet la validation du test.

Les produits de l'invention à une dose de 10 mg/kg-1 permettent une inhibition de l'augmentation du volume de la patte dans la phase retardée de l'inflammation (Jours 14 à 18).

### EXEMPLE H : COMPOSITION PHARMACEUTIQUE DESTINEE AU TRAITEMENT DES TROUBLES DU SYSTEME NERVEUX CENTRAL

Comprimés dosés à 0,1 mg de 3-méthyl-6-[2-(4-benzylpipérazin-1-yl) éthyl]benzothiazolinone
Formule pour 10 000 comprimés :
- 3-méthyl 6-[2-(4-benzylpipérazin-1-yl)éthyl]benzothiazolinone 1 g
- Amidon de blé 75 g
- Amidon de maïs 75 g
- Lactose 325 g
- Stéarate de magnésium 10 g
- Silice 5 g
- Hydroxypropyl cellulose 10 g

### EXEMPLE I : COMPOSITION PHARMACEUTIQUE

Comprimés dosés à 0,1 mg de 6-(3-morpholinopropyl)benzothiazolinone.
Formule pour 10 000 comprimés :
- 6-(3-morpholinopropyl)benzothiazolinone 1 g
- Amidon de blé 75 g
- Amidon de maïs 75 g
- Lactose 325 g
- Stéarate de magnésium 10 g
- Silice 5 g
- Hydroxypropyl cellulose 10 g

## Revendications

1. Composés de formule (I) : 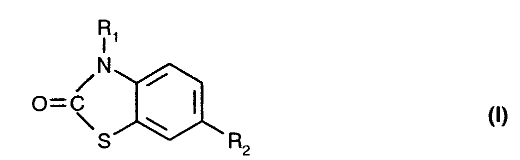 dans laquelle :
- R₁ représente un hydrogène ou un radical alkyle,
- et R₂ représente un groupement de formule R₂₁ : 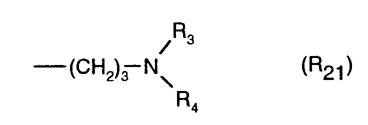
dans laquelle :
- R₃ et R₄ représentent indépendamment l'un de l'autre un atome d'hydrogène ou un radical choisi parmi alkyle, cycloalkyle, cycloalkylalkyle, alkényle, alkynyle, et -(CH₂)m aryle ; avec m représentant 0, 1, 2, 3 ou 4, le groupement aryle pouvant être non substitué ou substitué,
ou bien R₃ et R₄ forment ensemble un hétérocycle saturé de 5 à 9 sommets pouvant contenir d'autres hétéroatomes choisis parmi oxygène et soufre et pouvant être non substitué ou substitué ou un groupement 4-(méthoxyphényl)-pipérazin-1-yl,
ou groupement de formule R₂₂ : 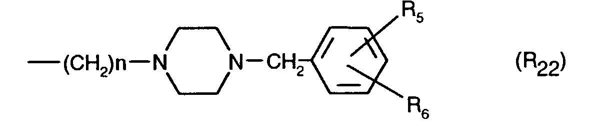 dans laquelle n représente 2,3 ou 4,
et R₅ et R₆, identiques, représentent un hydrogène ou un halogène,
étant entendu que lors de la description de la formule (I),
- le terme "substitué" associé au radical "aryle" signifie que la substitution peut se faire par un ou plusieurs radicaux choisis parmi halogène, hydroxy, alkyle, alkoxy inférieur et alkyle substitué par un ou plusieurs halogènes,
- le terme "substitué" associé à l'hétérocycle formé par -NR₃R₄ signifie que la substitution peut se faire par un ou plusieurs radicaux alkyle,
- les termes "alkyle" et "alkoxy" désignent des groupements linéaires ou ramifiés contenant de 1 à 6 atomes de carbone,
- les termes "alkényle" et "alkynyle" désignent des groupements linéaires ou ramifiés de 2 à 6 atomes de carbone,
- le terme "aryle" signifie phényle ou naphtyle,
- et le terme "cycloalkyle" représente un groupement cyclique de 3 à 8 atomes de carbone,
leurs isomères optiques et leurs sels d'addition à un acide pharmaceutiquement acceptable ou, lorsque R₁ représente un hydrogène, leurs sels d'addition à une base pharmaceutiquement acceptable.

2. Composé selon la revendication 1 qui est la 6-(3-morpholinopropyl)benzothiazolinone et ses sels d'addition à un acide pharmaceutiquement acceptable.

3. Composé selon la revendication 1 qui est la 3-méthyl 6-(3-morpholinopropyl) benzothiazolinone et ses sels d'addition à un acide pharmaceutiquement acceptable.

4. Composé selon la revendication 1 qui est la 3-méthyl 6-{2-[4-(2,4-dichlorobenzyl)pipérazin-1-yl]éthyl}benzothiazolinone et ses sels d'addition à un acide pharmaceutiquement acceptable.

5. Composé selon la revendication 1 qui est la 3-méthyl 6-{2-[4-(3,4-dichlorobenzyl)pipérazin-1-yl]éthyl}benzothiazolinone et ses sels d'addition à un acide pharmaceutiquement acceptable.

6. Composé selon la revendication 1 qui est la 3-méthyl 6-[4-(4-benzylpipérazin-1-yl)butyl]benzothiazolinone et ses sels d'addition à un acide pharmaceutiquement acceptable.

7. Procédé de préparation des composés de formule (I) selon l'une des revendication 1 à 6 dans lequel on introduit dans une solution appropriée un composé de formule (II) : 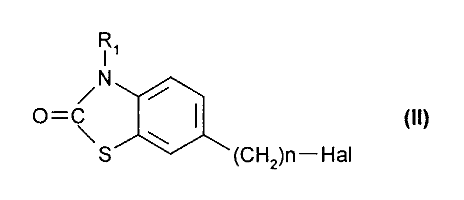 dans laquelle R₁ et n sont tels que définis dans la revendication 1 et Hal est un atome d'halogène, que l'on condense :
- soit avec un composé de formule (III/a) : 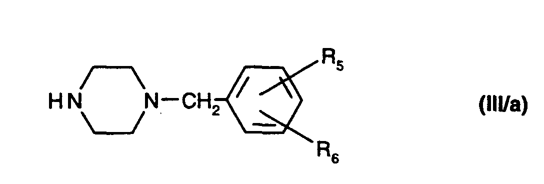 dans laquelle R₅ et R₆ sont tels que définis dans la revendication 1,
afin d'obtenir les composés de formule (I/a) : 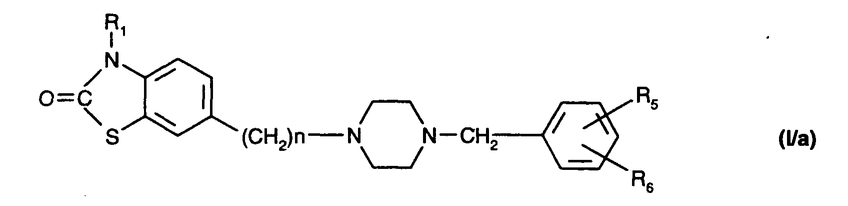 dans laquelle R₁, R₅, R₆ et n sont tels que définis précédemment,
- soit, lorsque n est égal à 3, avec un composé de formule (III/b) : 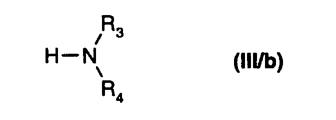 dans laquelle R₃ et R₄ sont tels que définis dans la revendication 1
pour obtenir un composé de formule (I/b) : 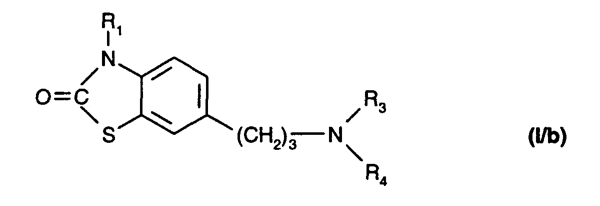 dans laquelle R₁, R₃ et R₄ sont tels que définis précédemment,
les composés de formule (I/a) et (I/b) formant l'ensemble des composés de formule (I), composés de formule (I) qui sont, si on le désire, séparés, le cas échéant, en leurs différents isomères optiques ou salifiés avec un acide pharmaceutiquement acceptable, ou si R₁ représente un atome d'hydrogène, salifiés par une base pharmaceutiquement acceptable.

8. Compositions pharmaceutiques contenant les composés de formule (I) selon l'une des revendication 1 à 6 ou un de leurs sels d'addition à un acide ou le cas échéant à une base pharmaceutiquement acceptable, en combinaison avec un ou plusieurs excipients.

9. Composition pharmaceutique selon la revendication 8 utile dans la prévention et le traitement des maladies liées aux récepteurs sigma.

10. Composition pharmaceutique selon l'une des revendications 8 ou 9 utile dans la prévention et le traitement des maladies psychiatriques, de la dépression, de l'anxiété et de l'arthrite aiguë ou chronique.

11. Composition pharmaceutique selon l'une des revendications 8 à 10 utile dans la prévention et le traitement des maladies psychiatriques.

## Claims

1. Compounds of formula (I) : 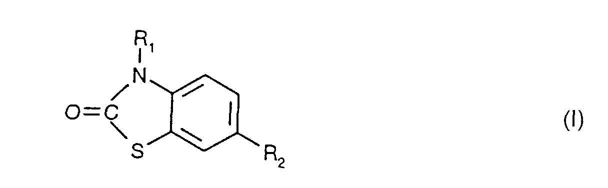 wherein :
- R₁ represents hydrogen or an alkyl radical,
- and R₂ represents a group of formula R₂₁ : 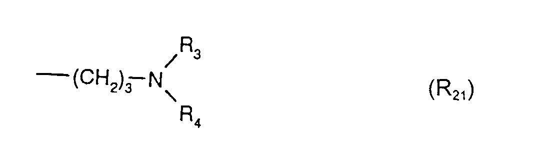
wherein
- R₃ and R₄ represent, independently of one another, a hydrogen atom or a radical selected from alkyl, cycloalkyl, cycloalkylalkyl, alkenyl, alkynyl and ―(CH₂)ₘ-aryl; m representing 0, 1, 2, 3 or 4 and it being possible for the aryl group to be unsubstituted or substituted,
or R₃ and R₄ together form a saturated heterocycle having from 5 to 9 ring members, it being possible for the heterocycle to contain other hetero atoms selected from oxygen and sulphur and it being possible for the heterocycle to be unsubstituted or substituted, or together form a 4-(methoxyphenyl)piperazin-1-yl group,
or a group of formula R₂₂ : 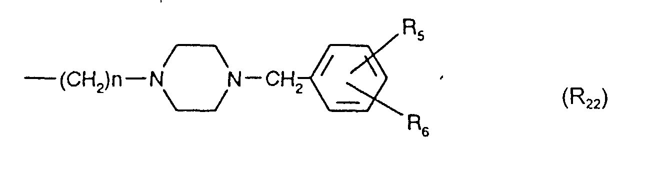 wherein n represents 2, 3 or 4,
and R₅ and R₆, which are identical or different, represent hydrogen or halogen,
wherein, in the description of formula (I),
- the term "substituted" associated with the "aryl" radical denotes that the substitution may be made by one or more radicals selected from halogen, hydroxy, alkyl, lower alkoxy and alkyl substituted by one or more halogen atoms,
- the term "substituted" associated with the heterocycle formed by -NR₃R₄ denotes that the substitution may be made by one or more alkyl radicals,
- the terms "alkyl" and "alkoxy" denote linear or branched groups containing from 1 to 6 carbon atoms,
- the terms "alkenyl" and "alkynyl" denote linear or branched groups containing from 2 to 6 carbon atoms,
- the term "aryl" denotes phenyl or naphthyl,
- and the term "cycloalkyl" denotes a cyclic group containing from 3 to 8 carbon atoms,
their optical isomers, and addition salts thereof with a pharmaceutically acceptable acid or, when R₁ represents a hydrogen atom, addition salts thereof with a pharmaceutically acceptable base.

2. Compound according to claim 1 that is 6-(3-morpholinopropyl)benzothiazolinone and addition salts thereof with a pharmaceutically acceptable acid.

3. Compound according to claim 1 that is 3-methyl-6-(3-morpholinopropyl)benzothiazolinone and addition salts thereof with a pharmaceutically acceptable acid.

4. Compound according to claim 1 that is 3-methyl-6-{2-[4-(2,4-dichlorobenzyl)piperazin-1-yl]ethyl}benzothiazolinone and addition salts thereof with a pharmaceutically acceptable acid.

5. Compound according to claim 1 that is 3-methyl-6-{2-[4-(3,4-dichlorobenzyl)piperazin-1-yl]ethyl}benzothiazolinone and addition salts thereof with a pharmaceutically acceptable acid.

6. Compound according to claim 1 that is 3-methyl-6-[4-(4-benzylpiperazin-1-yl)butyl]benzothiazolinone and addition salts thereof with a pharmaceutically acceptable acid.

7. Process for the preparation of compounds of formula (I) according to any one of claims 1 to 6, wherein there is introduced into an appropriate solution a compound of formula (II) : 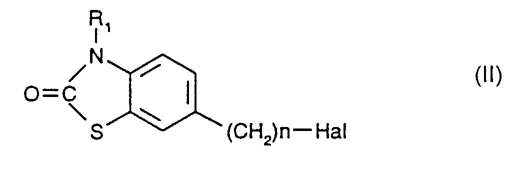 wherein R₁ and n are as defined in claim 1 and Hal is a halogen atom, which is condensed:
- either with a compound of formula (III/a) : 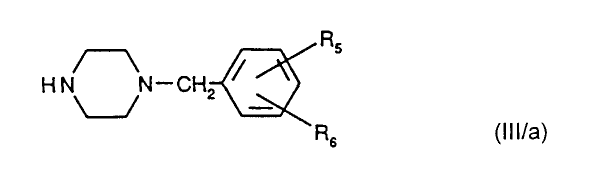 wherein R₅ and R₆ are as defined in claim 1
to obtain compounds of formula (I/a) : 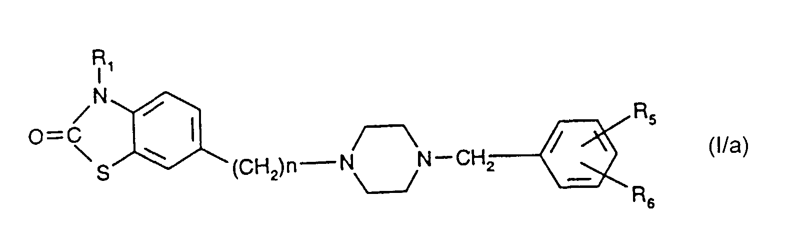 wherein R₁, R₅, R₆ and n are as defined hereinbefore,
- or, when n is 3, with a compound of formula (III/b) : 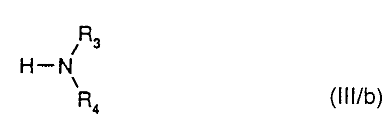 wherein R₃ and R₄ are as defined in claim 1,
to obtain a compound of formula (I/b): 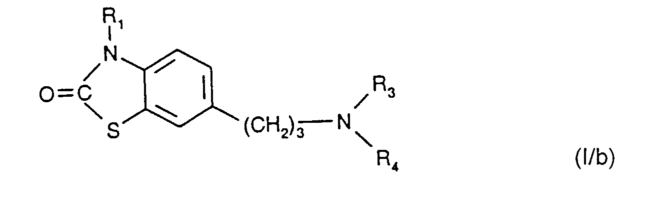 wherein R₁, R₃ and R₄ are as defined hereinbefore,
the compounds of formulae (I/a) and (I/b) constituting the totality of the compounds of formula (I), which compounds of formula (I), if desired, are separated, where appropriate, into their different optical isomers or are converted into salts with a pharmaceutically acceptable acid or, when R₁ represents a hydrogen atom, are converted into salts with a pharmaceutically acceptable base.

8. Pharmaceutical compositions comprising compounds of formula (I) according to any one of claims 1 to 6, or an addition salt thereof with a pharmaceutically acceptable acid or, where appropriate, a pharmaceutically acceptable base, in combination with one or more excipients.

9. Pharmaceutical composition according to claim 8, for use in the prevention and treatment of disorders associated with sigma receptors.

10. Pharmaceutical composition according to either claim 8 or claim 9, for use in the prevention and treatment of psychiatric disorders, depression, anxiety and acute or chronic arthritis.

11. Pharmaceutical composition according to any one of claims 8 to 10, for use in the prevention and treatment of psychiatric disorders.

## Patentansprüche

1. Verbindungen der Formel (I): 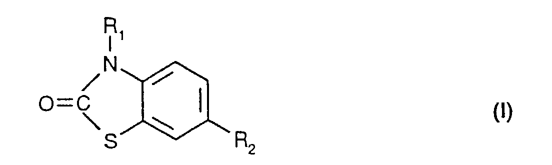 in der:
- R₁ Wasserstoff oder eine Alkylgruppe bedeutet,
- und R₂ eine Gruppe der Formel R₂₁: 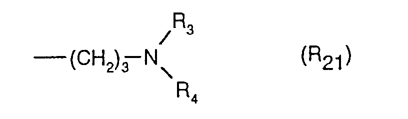
in der:
- R₃ und R₄ unabhängig voneinander Wasserstoffatome oder Gruppen ausgewählt aus Alkyl, Cycloalkyl, Cycloalkylalkyl, Alkenyl, Alkinyl und -(CH₂)ₘ-Aryl; worin m 0, 1, 2, 3 oder 4 darstellt und die Arylgruppe unsubstituiert oder substituiert sein kann, bedeuten,
oder R₃ und R₄ gemeinsam einen gesättigten Heterocyclus mit 5 bis 9 Kettengliedern, der andere Heteroatome ausgewählt aus Sauerstoff und Schwefel enthalten und unsubstituiert oder substituiert sein kann, oder eine 4-(Methoxyphenyl)-piperazin-1-yl-gruppe bilden,
oder eine Gruppe der Formel R₂₂ bedeutet: 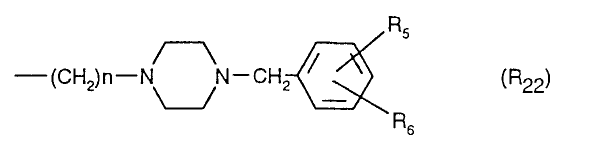 in der n 2, 3 oder 4 darstellt,
und R₅ und R₆, die identisch sind, Wasserstoff oder Halogene bedeuten, mit der Maßgabe, daß bei der Definition der Bedeutungen der Formel (I),
- der Begriff "substituiert" bezogen auf den "Aryl"-Rest bedeutet, daß eine Substitution durch einen oder mehrere Reste ausgewählt aus Halogen, Hydroxy, Alkyl, Niedrigalkoxy und Alkyl substituiert durch ein oder mehrere Halogene vorliegen kann,
- der Begriff "substituiert" im Hinblick auf den den durch ―NR₃R₄ gebildeten Heterocyclus bedeutet, daß die Substitution durch einen oder mehrere Alkylreste erfolgen kann,
- dioe Begriffe "Alkyl" und "Alkoxy" für geradkettige oder verzweigte Gruppen mit 1 bis 6 Kohlenstoffatome stehen,
- die Begriffe "Alkenyl" und "Alkinyl" für geradkettige oder verzweigte Gruppen mit 2 bis 6 Kohlenstoffatomen stehen,
- der Begriff "Aryl" für Phenyl oder Naphthyl steht,
- und der Begriff "Cycloalkyl" für eine cyclische Gruppe mit 3 bis 8 Kohlenstoffatomen steht,
deren optische Isomeren und deren Additionssalze mit einer pharmazeutisch annehmbaren Säure oder, wenn R₁ Wasserstoff bedeutet, deren Additionssalze mit einer pharmazeutisch annehmbaren Base.

2. Verbindung nach Anspruch 1, nämlich 6-(3-Morpholinopropyl)-benzothiazolinon und dessen Additionssalze mit einer pharmazeutisch annehmbaren Säure.

3. Verbindung nach Anspruch 1, nämlich 3-Methyl-6-(3-morpholinopropyl)-benzothiazolinon und dessen Additionssalze mit einer pharmazeutisch annehmbaren Säure.

4. Verbindung nach Anspruch 1, nämlich 3-Methyl-6-{2-[4-(2,4-Dichlorbenzyl)-piperazin-1-yl]-ethyl}-benzothiazolinon und dessen Additionssalze mit einer pharmazeutisch annehmbaren Säure.

5. Verbindung nach Anspruch 1, nämlich 3-Methyl-6-{2-[4-(3,4-Dichlorbenzyl)-piperazin-1-yl]-ethyl}-benzothiazolinon und dessen Additionssalze mit einer pharmazeutisch annehmbaren Säure.

6. Verbindung nach Anspruch 1, nämlich 3-Methyl-6-[4-(4-benzylpiperazin-l-yl)-butyl]-benzothiazolinon und dessen Additionssalze mit einer pharmazeutisch annehmbaren Säure.

7. Verfahren zur Herstellung der Verbindungen der Formel (I) nach einem der Ansprüche 1 bis 6, gemäß dem man eine Verbindung der Formel (II): 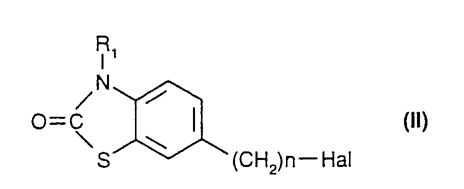 in der R₁ und n die in Anspruch 1 angegebenen Bedeutungen besitzen und Hal ein Halogenatom darstellt, in eine geeignete Lösung bringt und:
- entweder mit einer Verbindung der Formel (III/a): 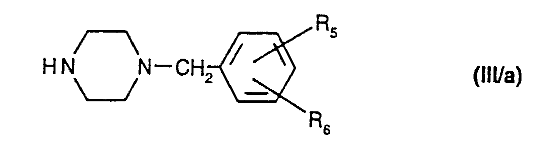 in der R₅ und R₆ die in Anspruch 1 angegebernen Bedeutungen besitzen, kondensiert zur Bildung der Verbindungen der Formel (I/a): 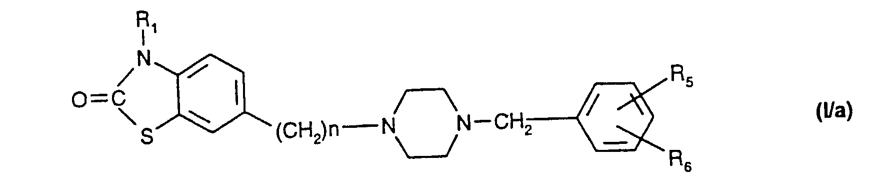 in der R₁, R₅, R₆ und n die oben angegebenen Bedeutungen besitzen,
- oder, wenn n den Wert 3 besitzt, mit einer Verbindung der Formel (III/b) kondensiert: 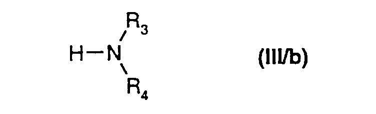 in der R₃ und R₄ die in Anspruch 1 angegebenen Bedeutungen besitzen, zur Bildung einer Verbindung der Formel (I/b): 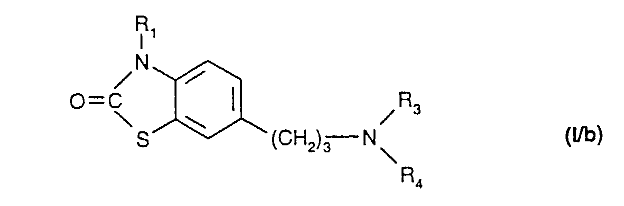 in der R₁, R₃ und R₄ die oben angegebenen Bedeutungen besitzen,
wobei die Verbindungen der Formeln (I/a) und (I/b) die Gesamtheit der Verbindungen der Formel (I) bilden, welche Verbindungen der Formel (I) gewünschtenfalls und gegebenenfalls in ihre verschiedenen optischen Isomeren aufgetrennt oder mit einer pharmazeutisch annehmbaren Säure oder, wenn R₁ ein Wasserstoffatom darstellt, mit einer pharmazeutisch annehmbaren Base in ihre Salze überführt werden.

8. Pharmazeutische Zubereitungen enthaltend die Verbindungen der Formel (I) nach einem der Ansprüche 1 bis 6 oder eines ihrer Additionssalze mit einer pharmazeutisch annehmbaren Säure oder gegebenenfalls einer pharmazeutisch annehmbaren Base in Kombination mit einem oder mehreren Trägermaterialien.

9. Pharmazeutische Zubereitung nach Anspruch 8 zur Vorbeugung und Behandlung von Erkrankungen, die mit Sigma-Rezeptoren verknüpft sind.

10. Pharmazeutische Zubereitung nach einem der Ansprüche 8 oder 9 zur Vorbeugung und Behandlung von psychiatrischen Erkrankungen, der Depression, der Angst und der akuten oder chronischen Arthritis.

11. Pharmazeutische Zubereitung nach einem der Ansprüche 8 bis 10 zur Vorbeugung und zur Behandlung von psychiatrischen Krankheiten.
